# EUROPÄISCHE PATENTSCHRIFT

(11) **EP 2 229 151 B1**
(45) Veröffentlichungstag und Bekanntmachung des Hinweises auf die Patenterteilung: **08.08.2012**
(21) Anmeldenummer: 08858493.3
(22) Anmeldetag: 10.12.2008
(51) Int. Cl.: A61K 9/20, A61K 9/14, A61K 47/24

(54) **SALZE VON WIRKSTOFFEN MIT POLYMEREN GEGENIONEN**
SALTS OF ACTIVE INGREDIENTS WITH POLYMERIC COUNTER-IONS
SELS DE PRINCIPES ACTIFS AYANT DES CONTRE-IONS POLYMÈRES

(30) Priorität: 12.12.2007 EP 07122995
(43) Veröffentlichungstag der Anmeldung: 22.09.2010
(73) Patentinhaber: BASF SE, 67056 Ludwigshafen (DE)
(72) Erfinder: KOLTER, Karl, 67117 Limburgerhof (DE); ANGEL, Maximilian, 67105 Schifferstadt (DE); MEYER-BÖHM, Kathrin, 90537 Feucht (DE)
(86) Internationale Anmeldenummer: PCT/EP2008/067228
(87) Internationale Veröffentlichungsnummer: WO 2009/074609

(56) Entgegenhaltungen:
- EP-A- 0 721 785
- EP-A- 0 821 965
- WO-A-2006/061700
- WO-A-2007/051743
- WO-A-2007/125028
- WO-A-2007/141182
- DE-A1- 19 637 479
- US-A- 5 219 563

## Beschreibung

Die vorliegende Erfindung betrifft feste Salze von Wirkstoffen mit polymeren Gegenionen.

Zahlreiche Arzneistoffe weisen eine sehr niedrige Wasserlöslichkeit auf und können dadurch nicht aus dem Magen- und Darmtrakt resorbiert werden. Die Folge ist eine sehr niedrige Bioverfügbarkeit. Bei Arzneistoffen, die eine basische oder saure Gruppe besitzen lassen sich durch Umsetzung mit Säuren oder Laugen entsprechende Salze bilden, die z. T. bessere Löslichkeiten aufweisen. Hierzu werden in der Regel niedermolekulare Säuren oder Laugen verwendet. Die gebräuchlichsten Säuren sind: Salzsäure, Schwefelsäure, Essigsäure, Citronensäure, Weinsäure, Fumarsäure, Maleinsäure, Malonsäure, Bernsteinsäure, Phosphorsäure. Als Basen finden NaOH, KOH, L-Lysin, L-Arginin, Triethanolamin oder Diethylamin Verwendung. Bei vielen Arzneistoffen sind jedoch auch die Salze mit diesen niedermolekularen Verbindungen schwer löslich in Wasser. Häufig besteht kaum ein Unterschied zwischen der Löslichkeit der Arzneistoffsäure oder -base und der eines Salzes mit den genannten Verbindungen. Die Ursache dieser schlechten Löslichkeit liegt in der Regel darin, dass das Salz ein sehr stabiles Kristallgitter ausbildet, das sich energetisch in einem günstigen Zustand befindet, wodurch die Tendenz in Lösung zu gehen niedrig ist. Falls zusätzlich der E-nergiegewinn durch die Hydratation niedrig ist, wird die Löslichkeit weiter herabgesetzt. Salze von Arzneistoffen mit polymeren Säuren oder Basen wurden bisher im Prinzip schon hergestellt, jedoch wurden Polymere eingesetzt, die nicht über einen großen pH-Bereich löslich waren - insbesondere nicht im physiologisch bedeutsamen Bereich von pH 2-8 - oder die in Lösung als Säure, Base oder Salz eine hohe Viskosität aufwiesen. Werden Polymere eingesetzt, die bei sauren pH-Werten, unlöslich sind - wie dies bei magensaftresistenten Polymeren der Fall ist - erfolgt keine Auflösung des Arzneistoffes, sondern eine Ausfällung des Polymers. Dadurch wird die Freisetzung verhindert oder zumindest stark verlangsamt. Es entsteht dadurch eine magensaftresistente Zubereitung, die die Bioverfügbarkeit herabsetzt, da einerseits im Magen keine Resorption erfolgen kann und andererseits im Dünndarm bei neutralen pH-Werten erst eine Auflösung der Zubereitung erfolgen muss, wodurch die Freisetzung relativ spät erfolgt und nicht mehr die gesamte Dünndarmoberfläche für die Resorption zur Verfügung steht.

Weisen die Polymere eine hohe Viskosität in wässriger Lösung auf, wird die Wirkstofffreisetzung aus einer festen Darreichungsform wie z.B. einer Tablette ebenfalls verzögert. Bei der Auflösung des Salzes entsteht an der Oberfläche der Tablette und in den Hohlräumen ein Gel bzw. eine hochviskose Lösung, die das weitere Vordringen des Wassers in den Tablettenkern behindert und den Zerfall verlangsamt. Durch diesen Effekt wie auch durch den verringerten Diffusionskoeffizienten der Arzneistoffmoleküle durch Areale mit hoher Viskosität wird die Freisetzung des Arzneistoffes verzögert. Diese Möglichkeit der Freisetzungsverzögerung macht man sich bei der Herstellung von Matrixretardformen mit Polymeren hoher Viskosität wie z.B. Alginaten, Xanthanen, Methylhydroxymethylcellulosen, Natriumcarboxymethylcellulosen, Pektinen etc. zu Nutze. In keinster Weise sind diese Polymeren aber zur Herstellung von schnellfreisetzenden Formen geeignet, bei denen ein schwerlöslicher Arzneistoff schnell aufgelöst werden soll und der gesamten Magen- und Dünndarmoberfläche zur Resorption dargeboten werden soll.

In EP 0211268 werden Minoxidilsalze mit polymeren Polyanionen beschrieben, die eine Freisetzungsverzögerung aufweisen und zur dermalen Applikation verwendet werden. Minoxidil ist ein Arzneistoff der 4 zur Salzbildung befähigte Gruppen enthält und die entsprechenden polymeren Salze waren schlechter löslich als das Hydrochlorid. Durch die zahlreichen zur Salzbildung befähigten Gruppen ist die Dissoziation des Salzes stark vermindert und die Löslichkeit gegenüber dem Hydrochlorid nicht verbessert. Orale Applikationen sind nicht beschrieben.

In US 4997643 wird ein biokompatibles, filmbildendes Delivery System für die topische Applikation beschrieben, das ein polymeres Salz mit einer carboxylgruppentragenden Komponente beinhaltet. Als Arzneistoff wird ebenfalls Minoxidil eingesetzt, der die o.g. Besonderheiten aufweist. Orale Anwendungen sind nicht beschrieben.

In US 4248855 werden flüssige Zubereitungen beansprucht, die Salze aus basischen Arzneistoffen und wasserunlöslichen Polymeren enthalten, und die einen Retardeffekt besitzen. Durch die Verwendung von wasserunlöslichen Polymeren zeigen die Zubereitungen keine schnelle Freisetzung und keine hohe Löslichkeit über einen großen pH-Bereich.

Aus der US 5736127 ist bekannt, dass aus basischen Arzneistoffen und Polymeren mit Carboxyl - Amidin - Carboxyl-Triaden Salze gebildet werden können. Aufgrund der hohen Molekulargewichte sind die Polymeren gelbildend, wodurch die Freisetzung der Wirkstoffe verzögert wird. Eine Eignung für schnell freisetzende Tabletten ist nicht gegeben.

In der US 4205060 werden Mikrokapseln mit verzögerter Freisetzung beschrieben, die im Kern ein Salz eines basischen Arzneistoffes mit einem carboxylgruppenhaltigem Polymer enthalten und der mit einem wasserunlöslichen Polymer umhüllt ist. Durch das carboxylgruppenhaltige Polymer wird die Freisetzung der verwendeten löslichen Arzneistoffe vermindert.

Salze des Ranitidins mit Polycarbonsäuren sind in EP 0721785 beschrieben. Die Polycarbonsäuren binden das Ranitidin und sollen dessen Bitterkeit herabsetzen. Allerdings sind niedermolekulare Salze des Ranitidin gut löslich, so dass die Polycarbonsäuren lediglich die Beweglichkeit und Diffusion des Ranitidins einschränken, so dass es nicht so schnell an die Bitterrezeptoren gelangt.

WO 2007/051743 betrifft die Verwendung von Copolymeren, die durch Polymerisation von Vinylacetat und N-Vinyllactamen in Gegenwart eines Polyethers erhalten werden, als Solubilisatoren für in Wasser schwerlösliche Substanzen sowie die entsprechenden Zubereitungen.

Aufgabe der vorliegenden Erfindung war es, Wirkstoffsalze zu finden, die nach der Verarbeitung zu oralen Dosierungsformen eine im Vergleich zum entsprechenden Hydrochloridsalz schnellere Freisetzung des Wirkstoffs ermöglichen. Weiterhin sollten die gesuchten Wirkstoffsalze auch pH-unabhängig löslich sein. Aufgabe war es weiterhin, solche Salze zu finden, die eine gute Tablettierbarkeit des Wirkstoffs ermöglichen.

Demgemäß wurden wasserlösliche Salze von Arzneistoffen mit polymeren Gegenionen gemäβ Anspruch 1 bereitgestellt.

Weiterhin wurden Verfahren zu ihrer Herstellung und Verarbeitung zu festen Darreichungsformen gefunden.

Als Polymere mit Anioncharakter werden nicht-gelbildende Polymere verwendet.

Das Polymer mit Anioncharakter weist bevorzugt einen Anteil von mindestens 50 Gew.-% Acrylsäure und/oder Methacrylsäure auf.

Als Wirkstoffe mit Kationcharakter werden erfindungsgemäß Wirkstoffbasen bezeichnet. Bevorzugte Wirkstoffe mit Kationcharakter sind solche mit mindestens einer und höchstens zwei zur Salzbildung befähigten Gruppen.

Die jeweiligen polymeren Gegenionen werden so gewählt, dass das polymere Arzneistoffsalz eine höhere Wasserlöslichkeit als der Arzneistoff und das entsprechende Hydrochlorid des Arzneistoffes besitzt.

Durch die beschriebene Salzbildung lassen sich auch Arzneistoffe in Lösung bringen, bei denen weder die neutrale Form noch die entsprechenden niedermolekularen Salze wasserlöslich sind. Bei diesen Arzneistoffen ist dann die Auflösung im Magen- und Darmtrakt sehr langsam und somit limitierend für die Resorption, wodurch häufig eine niedrige Bioverfügbarkeit resultiert (gemäß dem Biopharmaceutical Classification System: Wirkstoffe der Klasse II (Amidon et al., Pharm. Res. 12, 413-420)). Die Erfindung betrifft Arzneistoffe, die in nicht geladener Form oder als Hydrochlorid Löslichkeiten kleiner 0, 1 % (m/m) in Wasser, künstlichem Darmsaft oder Magensaft aufweisen.

Voraussetzung für eine über einen breiten pH-Bereich erheblich erhöhte Löslichkeit ist, dass die verwendeten Polymere ebenfalls über einen breiten pH-Bereich wasserlöslich sind. Nur so kommt es bei pH-Wertveränderungen nicht zu Polymerausfällungen. Die Polymere sollen auch nicht gelbildend sein.

Um die Auflösung des Wirkstoffes aus dem polymeren Salz nicht zu behindern, sollte die Viskosität des verwendeten Polymers niedrig sein. Vorteilhaft haben sich Polymere erwiesen, die in 5 gew.-%iger Lösung Viskositäten kleiner 100mPas, bevorzugt kleiner 75mPas und besonders bevorzugt kleiner 50mPas aufweisen. Niedrige Viskositäten bedingen in der Regel niedrige Molekulargewichte. Vorzugsweise werden nichtvernetzte Polymere verwendet. Aus praktischen Gründen wird statt des Molekulargewichts häufig der K-Wert ermittelt. Dabei handelt es sich um Polymere mit einem K-Wert < 90, besonderes bevorzugt < 50, insbesonder < 40 (gemessen 1 gew.-%ig in Wasser).

Die Glasübergangstemperatur der Polymeren sollte oberhalb 60°C liegen, damit die polymeren Salze gut zu Tabletten verarbeitet werden können und es in der Stabilitätsprüfung bei leicht erhöhten Temperaturen zu keinen Veränderungen kommt.

Die Polymere mit Anioncharakter können Homopolymere, Copolymere, Blockcopolymere, gepfropfte Copolymere, Kamm- oder Sterncopolymere sein.

Zur Herstellung der Polymeren mit Anioncharakter können Monomere, die saure Gruppen wie Carboxylgruppen, Sulfonsäuregruppen, Phosphorsäuregruppen oder Phosphonsäuregruppen tragen (im Folgenden auch "saure Monomere" genannt), verwendet werden.

Der Anteil an sauren Monomeren beträgt bevorzugt mehr als 80 Gew.% und besonders bevorzugt mehr als 90 Gew.%, insbesondere mehr als 98 Gew.-%. Dabei beträgt der Anteil an carboxylgruppentragenden Monomeren an der Gesamtmenge aller Monomere mindestens 40 Gew.-%, davon sind bevorzugt mindestens 50 Gew.-% Acrylsäure und/oder Methacrylsäure.

Grundsätzlich geeignete Monomere dieser Gruppen sind:
Sulfonsäuregruppen enthaltende Monomere wie Vinylsulfonsäure, Methylallylsulfonsäure, Styrolsulfonsäure, Toluolsulfonsäure, 2-Sulfoethylmethacrylat, 2-Acrylamido-2-methylpropansulfonsäure, 3-Sulfoethylpropylacrylat oder Allylsulfonsäure
Phosphonsäure- oder Phosphorsäure-Gruppen tragende Monomere wie Vinylphosphonsäure, Vinyldiphosphonsäure, 2-(Methacryloyloxy)ethyl-phosphorsäure, 2-(Acryloyloxy)ethyl-phosphorsäure,
Carboxylgruppentragende Monomere wie Acrylsäure, Methacrylsäure, Maleinsäure, Maleinsäureanhydrid, Crotonsäure,4-Oxo-2-pentensäure, 1,6-Hexandicarbonsäuremonovinylester, Hydroxy[(1-oxo-2-propenyl)amino]essigsäure,1,4-Butandicarbonsäure-mono[2-[(2-methyl-1-oxo-2-propenyl)oxy]ethyl]ester, Acrylsäure-2-carboxyethylester, 1,4-Butandicarbonsäuremonovinylester, 6-[(2-methyl-1-oxo-2-propenyl)amino]hexansäure, Methacrylsäure-2-carboxypropylester, 2-Methyl-3-[(2-methyl-1-oxo-2-propenyl)amino]-1-propansulfonsäure, 2-Methyl-2-pentensäure.

Es können auch mehrere saure Monomere miteinander kombiniert werden.

Bevorzugte Sulfonsäure-Monomere sind 2-Sulfoethylmethacrylat, 3-Sulfopropylacrylat , 2- Acrylamido-2-methylpropansulfonsäure und Allylsulfonsäure.

Besonders bevorzugt sind als carboxylgruppen-tragende Monomere Acrylsäure, Methacrylsäure, Maleinsäure oder Maleinsäureanhydrid.

Besonders bevorzugt sind neben den Homopolymeren der Acrylsäure die Copolymere aus Acrylsäure und Maleinsäure sowie Copolymere aus Acrylsäure und Methacrylsäure oder Copolymere der Acryl- oder Methacrylsäure mit Itaconsäure. Itaconsäure kann dabei in Mengen von 1 bis 20 Gew.-%, bevorzugt 3 bis 15 Gew.-%, bezogen auf das Gesamtgewicht der Monomere, copolymerisiert werden.

Besonders bevorzugt sind weiterhin Copolymere der Acrylsäure oder Methacrylsäure mit Sulfonsäure-Monomeren, insbesondere Copolymere der Acrylsäure oder der Methacrylsäure mit 2-Acrylamido-2-methyl-propansulfonsäure oder 2-Sulfoethylmethacrylat. Solche Copolymere können 1 bis 20, bevorzugt, 2 bis 15, besonders bevorzugt 5 bis 10 Gew.-% an Sulfonsäure-Monomeren enthalten.

Die genannten sauren Monomere können zusammen mit neutralen Comonomeren polymerisiert werden. Die neutralen Monomere können wasserlöslich oder wasserunlöslich sein. Im Falle einer Wasserunlöslichkeit ist der Monomeranteil dadurch begrenzt, dass das Polymer irgendwann wasserunlöslich wird.

Geeignete neutrale Comonomere sind:
Ethylen, Propylen, Butylen, Styrol, Methylvinylether, Ester der Acrylsäure mit C1 - C20 Alkoholen, Ester der Methacrylsäure mit C1 - C20 Alkoholen, Vinylester von C1 - C20 Carbonsäuren.

Weiterhin eignen sich Hydroxyethylacrylat, Hydroxyethylmethacrylat, Hydroxypropylacrylat, Hydroxypropylmethacrylat, (Meth-)Acrylate mit 5 bis 50 Ethylenoxideinheiten, insbesondere den entsprechenden endgruppenverschlossenen Verbindungen (z.B. Methylendgruppe).

Besonders geeignet sind Copolymere aus sauren und neutralen Monomeren, die als neutrale Monomere Monomere mit substituierten Amidgruppen wie z. B. N,N- Dimethylvinylformamid, N,N- Dimethylvinylacetamid, N,N- Dimethylacrylamid, N,N-Diethylacrylamid, N,N- Dimethylmethacrylamid, Diethylmethacrylamid, enthalten. Ganz besonders geeignete Comonomere mit substituierten Amidgruppen sind die N-Vinylamide wie N-Vinylpyrrolidon, Vinylcaprolactam, Acrylamid, Methacrylamid, insbesondere Acrylamid.

Der Anteil an neutralen Monomeren kann 1 bis 60 Gew,.%, bevorzugt 1 bis 20, besonders bevorzugt 3 bis 15 Gew.-%, bezogen auf die Gesamtmenge der Monomeren betragen.

Besonders bevorzugt sind auch Copolymere aus zwei unterschiedlichen sauren Monmeren und zusätzlich neutralen Monomeren, insbesondere Copolymere der Acrylsäure oder der Methacrylsäure mit Sulfonsäure-Monomeren und N-Vinylamid-Monomeren.

Die Herstellung der erfindungsgemäßen Polymere kann auf an sich übliche Weise durch radikalische Polymerisation erfolgen. Vorzugsweise erfolgt die Polymerisation als Lösungspolymerisation in Wasser. Solche Verfahren sind dem Fachmann an sich bekannt. Geeignete Initiatoren sind beispielsweise Eisen(II)sulfat, Natriumperoxodisulfat oder Wasserstoffperoxid.

Es kann sich auch empfehlen, die Polymerisation in Gegenwart von Reglern, beispielsweise von Natriumhypophosphit, durchzuführen.

Die Polymerisation kann kontinuierlich oder als Batch-Verfahren erfolgen, bevorzugt werden die polymere über Zulaufverfahren erhalten.

Die Überführung der Polymerlösungen in die feste Form kann ebenfalls nach üblichen Verfahren durch Sprühtrocknung, Gefriertrocknung oder Walzentrocknung erfolgen.

Bei der Herstellung der erfindungsgemäßen Polymeren ist vorzugsweise darauf zu achten, dass diese keine niedermolekulare Anionen wie z. B. Chlorid, Sulfat, etc. aufweisen, die zu schwerlöslichen Salzen mit Wirkstoffen führen können.

Es kann sich jedoch von Fall zu Fall empfehlen, die erfindungsgemäßen polymeren Salze auch im Gemisch mit schwerer löslichen niedermolekularen Salzen der Wirkstoff oder auch im Gemisch mit den entsprechenden teilneutralisierten polymeren Salzen zu verwenden.

Prinzipiell sind alle pharmazeutischen Wirkstoffe, Nutraceuticals, Lebensmittelzusatzstoffe und auch Pflanzenschutzwirkstoffe geeignet, die eine für eine Salzbildung ausreichende Basizität aufweisen. Für sehr schwach basische Wirkstoffe sind somit stärkere Säuren wie Sulfonsäuren geeignet, für mittel bis stark basische Wirkstoffe können auch Carbon- oder Phosphonsäuren verwendet werden. Der pKa-Wert des sauren Polymers muss gleich oder kleiner dem pKa-Wert des basischen Wirkstoffes sein. Ist dies nicht der Fall, dann ist das Salz nicht stabil gegen Hydrolyse. Besonders vorteilhaft ist, wenn zwischen den pKa-Werten der Polymeren und der Wirkstoff ein Unterschied von mindestens 2 Einheiten vorliegt.

Die Herstellung der erfindungsgemäßen Salze kann prinzipiell durch Trocknungs-, Schmelz- oder Ausfällungsprozesse erfolgen.

### Trocknung einer wässrigen oder organischen Lösung

Der Wirkstoff und das Polymer werden in Wasser oder organischen Lösungsmitteln gelöst und die Lösung wird anschließend getrocknet. Das Lösen kann auch bei erhöhten Temperaturen (30 - 200°C) und unter Druck erfolgen.

Prinzipiell sind alle Trocknungsarten möglich, wie z.B. Sprühtrocknung, Wirbelschichttrocknung, Schaufeltrocknung, Gefriertrocknung, Vakuumtrocknung, Bandtrocknung, Walzentrocknung, Schleppgastrocknung, Eindampfen etc..

### Schmelzprozesse

Der Wirkstoff wird mit dem Polymer gemischt. Durch Erhitzen auf Temperaturen von 50 - 250°C erfolgt die Herstellung des polymeren Salzes. Hierbei sind Temperaturen oberhalb der Glasübergangstemperatur des Polymers oder des Schmelzpunktes des Wirkstoffes vorteilhaft. Durch Zugabe eines weichmachenden Hilfsstoffes wie z. B. Wasser, organisches Lösungsmittel, übliche organische Weichmacher lässt sich die Verarbeitungstemperatur entsprechend absenken. Besonders vorteilhaft sind hier Hilfsstoffe, die nachher recht einfach wieder abgedampft werden können, d.h. einen Siedepunkt unter 180°C bevorzugt unter 150°C aufweisen.

Vorzugsweise kann diese Art der Herstellung in einem Extruder vorgenommen werden.

Der Extruder ist bevorzugt ein gleichläufiger Zweischneckenextruder. Die Schneckenkonfiguration kann dabei je nach Produkt unterschiedlich stark scherend sein. Es empfiehlt sich insbesondere in der Aufschmelzzone Knetelemente einzusetzen. Dabei können auch rückläufige Knetelemente eingesetzt werden. Nach der Aufschmelzzone kann eine Entgasungszone nachgeschaltet werden, die vorteilhafterweise bei Atmosphärendruck betrieben wird, gewünschtenfalls aber auch unter Vakuum betrieben werden kann.

Der Produktaustrag kann über Runddüsen mit Durchmessern von 1 bis 5, vorzugsweise 2 bis 3 mm, erfolgen. Die extruder können auch mit Düsenplatten ausgerüstet sein. Andere Düsenformen, wie Schlitzdüsen können ebenfalls eingesetzt werden, vor allem dann wenn ein größerer Materialdurchsatz angestrebt wird.

Die Extruder sind üblicherweise mit beheizbaren Zylindern ausgerüstet. Die resultierende Produkttemperatur ist jedoch vom Schergrad des eingesetzten Schneckenelements abhängig und kann mitunter 20-30°C höher sein als die eingestellte Zylindertemperatur.

Üblicherweise eignen sich Extruder mit einer Länge von 25D bis 40D.

Prinzipiell können auch mehrere Wirkstoffbasen mit dem Polymer im Extruder zum Salz umgesetzt werden.

Die Aus dem Extruder austretenden Extrudatstränge können auf an sich bekannte weise, beispielsweis durch Abschlagtechniken zerkleinert werden.

Die resultierenden Stränge können mit einem Granulator zu Granulatkörnern verarbeitet werden und diese können wiederum weiter zu einem Pulver zerkleinert (gemahlen) werden. Das Granulat oder Pulver kann in Kapseln gefüllt oder unter Anwendung üblicher Tablettierhilfsstoffe zu Tabletten verpresst werden.

In einer besonderen Ausführung der Extrusion kann zunächst das Polymer dem Extruder zugeführt werden und geschmolzen werden. Über eine zweite Eingabestelle wird dann die Wirkstoffbase zugegeben. Über weitere Einzugsstellen lassen sich zusätzliche Wirkstoffbasen hinzudosieren.

Ferner ist es möglich, während der Extrusion Wasser, organische Lösungsmittel, Puffersubstanzen oder Weichmacher einzusetzen, um die Umsetzung der Wirkstoffbase mit dem sauren Polymer zu beschleunigen. Insbesondere Wasser oder flüchtige Alkohole bieten sich hierfür an. Dieses Verfahren ermöglicht die Umsetzung bei niedrigerer Temperatur. Die Lösungsmittel- und/oder Weichmachermengen liegen üblicherweise zwischen 0 und 30% der extrudierbaren Masse. Das Wasser oder Lösungsmittel kann schon durch eine Entgasungsstelle im Extruder bei Normaldruck oder durch Anlegen von Vakuum entfernt werden. Alternativ verdampfen diese Komponenten, wenn das Extrudat den Extruder verlässt und der Druck sich auf Normaldruck reduziert. Im Falle von schwerer flüchtigen Komponenten kann das Extrudat entsprechend nachgetrocknet werden.

Zwei weitere Ausführungsformen bestehen darin, dass entweder der Wirkstoff in einem Lösungsmittel gelöst wird und dem Polymer im Extruder zugeführt wird oder dass das Polymer in einem Lösungsmittel gelöst wird und dem Wirkstoff zugeführt wird.

Gemäß einer Ausführungsform des Herstellungsverfahrens wird direkt im Anschluß an die Extrusion die thermoplastische Masse zu einem tablettenähnlichen Komprimat kalandriert, die die endgültige Darreichungsform darstellt. In dieser Variante kann es sinnvoll sein, weitere Bestandteile wir beispielsweise Polymere zur Einstellung der Glasübergangstemperatur und der Schmelzviskosität, Sprengmittel, Solubilisatoren, Weichmacher, freisetzungsbeeinflussende Hilfsstoffe, Retardierungsmittel, magensaftresistente Polymere, Farbstoffe, Geschmacksstoffe, Süßstoffe und weitere Zusatzstoffe. schon vor oder während der Extrusion zuzusetzen. Prinzipiell können diese Stoffe auch verwendet werden, wenn das Extrudat zunächst zerkleinert und danach zu Tabletten verpresst wird.

Der Wassergehalt der extrudierten Produkte beträgt in der Regel unter 5 Gew.-%.

Die Wasserlöslichkeit der Polymeren mit Anioncharakter im pH-Bereich 3-11 größer 10% (m/m) beträgt.

Durch den Zusatz von thermoplastischen Retardierungsmitteln wie Polyvinylacetat-Homopolymeren oder Formulierungen solcher Polyvinylacetat-Homopolymeren, weiterhin als Retardpolymeren auf Acrylatbasis bekannten Eudragit®-Typen RS, RL oder NE oder NM ,lässt sich die Freisetzung verlangsamen. Auf diese Weise können verlässlich freisetzende Retardformen schwerlöslicher Arzneistoffe hergestellt werden. Prinzipiell können die Umsetzungen zur Salzbildung auch in einem Mischer als Schmelzgranulation oder durch Befeuchtung mit Wasser oder einem Lösungsmittel als Feuchtgranulation durchgeführt werden. Granulate im weiteren Sinn sind auch mittels der Feuchtextrusion mit anschließender Sphäronisation herstellbar. Die resultierenden Pellets lassen sich dann als multiple dosage form in Hartkapseln abfüllen.

### Ausfällungsprozesse

Der Wirkstoff und das Polymer werden in Wasser oder einem organischen Lösungsmittel gelöst und anschließend wird schnell die Temperatur abgesenkt oder ein mit Wasser oder dem organischen Lösungsmittel mischbares Nichtlösungsmittel hinzugegeben. Dabei kommt es zur Ausfällung des polymeren Salzes, das dann abfiltriert oder abzentrifugiert wird und getrocknet wird. Geeignete Nichtlösungsmittel sind beispielsweise Aceton, Isopropanol oder n-Butanol.

Die beschriebenen polymeren Salze sind immer amorph, von daher besteht nicht das Problem wie bei niedermolekularen Salzen, dass der amorphe Zustand durch äußere Einflüsse oder im Laufe der Lagerung in einen thermodynamisch stabilen kristallinen Zustand übergehen kann. Der amorphe Zustand der erfindungsgemäßen Salze ist somit ein thermodynamisch stabiler Zustand, weil es für solche Stoffe keinen energieärmeren gibt. Somit unterscheiden sich die polymeren Salze prinzipiell von den niedermolekularen Salzen.

Ferner tritt bei niedermolekularen Salzen häufig das Problem auf, dass sie schlecht kristallisieren bzw. mit niedrigem Schmelzpunkt anfallen. Dieses Problem wird bei den beschriebenen polymeren Salzen vermieden.

Die erfindungsgemäßen polymeren Salze aus einer polymeren Anion-Komponente und einem Wirkstoff, der als basischen Kation-Komponente dient, besitzen eine ausgezeichnete Verarbeitbarkeit zu Darreichungsformen, insbesondere hinsichtlich der-Tablettierbarkeit. Daher lassen sich Tabletten mit einem Durchmesser von 10mm und 300mg Gewicht herstellen, die eine Bruchfestigkeit von über 200N besitzen. Das polymere Anion wirkt daher gleichzeitig als Bindemittel und verleiht der Tablettenrezeptur eine enorme Plastizität. Hingegen sind niedermolekulare Salze häufig sehr spröde und schlecht tablettierbar.

Die erfindungsgemäßen polymeren Salze eignen sich zur Löslichkeitsverbesserung von allen in Wasser wenig, schwer löslichen oder unlöslichen Wirkstoffen, die on der Lage sind mit den sauren Gruppen der Polymerkomponente Salze zu bilden. Gemäß DAB 9 (Deutsches Arzneimittelbuch) erfolgt die Einstufung der Löslichkeit pharmazeutischer Wirkstoffe wie folgt: wenig löslich (löslich in 30 bis 100 Teilen Lösungsmittel); schwer löslich (löslich in 100 bis 1000 Teilen Lösungsmittel); praktisch unlöslich (löslich in mehr als 10000 Teilen Lösungsmittel). Die Wirkstoffe können dabei aus jedem Indikationsbereich kommen.

Als Beispiele seien hier Antihypertensiva, Vitamine, Cytostatika - insbesondere Taxol, Anästhetika, Neuroleptika, Antidepressiva, Antibiotika, Antimykotika, Fungizide, Chemotherapeutika, Urologika, Thrombozytenaggregationshemmer, Sulfonamide, Spasmolytika, Hormone, Immunglobuline, Sera, Schilddrüsentherapeutika, Psychopharmaka, Parkinsonmittel und andere Antihyperkinetika, Ophthalmika, Neuropathiepräparate, Calciumstoffwechselregulatoren, Muskelrelaxantia, Narkosemittel, Lipidsenker, Lebertherapeutika, Koronarmittel, Kardiaka, Immuntherapeutika, regulatorische Peptide und ihre Hemmstoffe, Hypnotika, Sedativa, Gynäkologika, Gichtmittel, Fibrinolytika, Enzympräparate und Transportproteine, Enzyminhibitoren, Emetika, Abmagerungsmittel ,Durchblutungsfördernde Mittel, Diuretika, Diagnostika, Corticoide, Cholinergika, Gallenwegstherapeutika, Antiasthmatika, Broncholytika, Betarezeptorenblocker, Calciumantagonisten, ACE-Hemmer, Arteriosklerosemittel, Antiphlogistika, Antikoagulantia, Antihypotonika, Antihypoglykämika, Antihypertonika, Antifibrinolytika, Antiepileptika, Antiemetika, Antidota, Antidiabetika, Antiarrhythmika, Antianämika, Antiallergika, Anthelmintika, Analgetika, Analeptika, Aldosteronantagonisten oder antivirale Wirkstoffe oder Wirkstoffe zur Behandlung von HIV-Infektionen und des AID-Syndroms genannt.

Bei der Herstellung der erfindungsgemäßen festen Dosierungsformen können gegebenenfalls übliche pharmazeutische Hilfsstoffe mitverarbeitet werden. Dabei handelt es sich um Stoffe aus der Klasse der Füllstoffe, Weichmacher, Löslichkeitsvermittler, Bindemittel, Silikate sowie Spreng- und Adsorptionsmittel, Schmiermittel, Fließmittel, Farbstoffe, Stabilisatoren wie Antioxidantien, Netzmittel, Konservierungsmittel, Formentrennmittel, Aromen oder Süßstoffe, bevorzugt um Füllstoffe, Weichmacher und Löslichkeitsvermittler.

Als Füllstoffe können z.B. anorganische Füllstoffe wie Oxide von Magnesium, Aluminium, Silicium, Titan- oder Calciumcarbonat, Calcium- oder Magnesiumphosphate oder organische Füllstoffe wie Lactose, Saccharose, Sorbit, Mannit zugesetzt werden.

Als Weichmacher eignen sich beispielsweise Triacetin, Triethylcitrat, Glycerolmonostearat, niedermolekulare Polyethylenglykole oder Poloxamere.

Als Löslichkeitvermittler eignen sich grenzflächenaktive Substanzen mit einem HLB-Wert (HydrophilicLipophilicBalance) größer 11, beispielsweise mit 40 Ethylenoxid-Einheitten ethoxiliertes hydriertes Ricinusöl (Cremophor® RH 40), mit 35 EthylenoxidEinheiten ethoxiliertes Ricinusöl (Cremophor eL), Polysorbat 80, Poloxamere oder Natriumlaurylsulfat.

Als Schmiermittel können Stearate von Aluminium, Calcium, Magnesium und Zinn, sowie Magnesiumsilikat, Silikone und ähnliche verwendet werden.

Als Fließmittel können beispielsweise Talk oder kolloidales Siliciumdioxid eingesetzt werden.

Als Bindemittel eignet sich zum Beispiel mikrokristalline Cellulose.

Als Sprengmittel können quervernetztes Polyvinylpyrrolidon oder quervernetzte Natriumcarboxymethylstärke sein. Stabilisatoren können sein Ascorbinsäure oder Tocopherol.

Farbstoffe sind z.B. Eisenoxide, Titandioxid, Triphenylmethanfarbstoffe, Azofarbstoffe, Chinolinfarbstoffe, Indigotinfarbstoffe, Carotinoide, um die Darreichungsformen einzufärben, Opakisierungsmittel wie Titandiodid oder Talkum, um die Lichtdurchlässigkeit zu erhöhen und um Farbstoffe einzusparen.

Die erfindungsgemäßen polymeren Salze von Wirkstoffen lassen sich ausgezeichnet granulieren und zu Tabletten verpressen, die aufgrund der hohen Löslichkeit in wässrigen Medien eine äußerst schnelle Wirkstofffreisetzung zeigen wird. Durch die verbesserte Löslichkeit wird eine erheblich verbesserte Bioverfügbarkeit erzielt. Die Löslichkeit beträgt üblicherweise 0.05 bis 5 % (Gew.-Teile Arzneistoff/ Gew.-Teile Wasser). Zudem ist die Bioverfügbarkeit viel reproduzierbarer, d.h. es gibt geringere interindividuelle Schwankungen.

Die erfindungsgemäßen Salze lassen sich zu vielen verschiedenen Darreichungsformen formulieren, wie z. B. Tabletten, Kapseln, Granulate, Pulver, Drug Delivery Systeme, Lösungen, Suppositorien, transdermale Systeme, Cremes, Gele, Lotionen, Injektionslösungen, Tropfen, Säfte, Sirupe,

### Beispiele

### Polymer A

Polyacrylsäure (MG 4000; K-Wert 11)

### Polymer B

Maleinsäure-Acrylsäure (Gewichtsverhältnis 50/50) Copolymer (MG 3000; K-Wert 10)

### Polymer C

Methacrylsäure- Acrylsäure ( Gewichtsverhältnis 70/30) Copolymer ( MG 20.000; K-Wert 12)

### Polymer D

Polyacrylsäure (MG 100.000; K-Wert 38.6)

### Polymer E

Acrylsäure /2-Sulfoethylmethacrylat Gewichtsverhältnis 90/10, K-Wert 82

### Polymer F

Acrylsäure / Itaconsäure Gewichtsverhältnis 90/10, K-Wert 55

### Polymer G

Acrylsäure/ 2-Acrylamido-2-methylpropansäure/ Acrylamid Gewichtsverhältnis 80/10/10, K-Wert 74

### Polymer H

Acrylsäure/2-Acrylamido-2-methylpropansäure Gewichtsverhältnis 98.2/10.8, K-Wert 82

Alle K-Werte wurden an 1 Gew-% igen wässrigen Lösungen bestimmt.

Der Zerfall der Tabletten wurde mit einem Erweka-Zerfallstester durchgeführt. Die Bestimmung der Freisetzung erfolgte nach der Pharm.Eur. 6.0.

Als Crospovidon wurde Kollidon CL-F, Fa. BASF AG, mittlere Teilchengröße 30 µm, verwendet.

Als Zweischneckenextruder für die Herstellung von Extrudaten wurde ein Extruder des Typs ZSK 25 der Firma Werner &Pfleiderer mit einem Schneckendurchmesser von 16mm und eine Länge von 25D eingesetzt. Die Temperatur der unterschiedlich beheizbaren Zylinder wurde bis auf den gekühlten Einlasszylinder konstant je nach Produkt eingestellt. Die eingesetzten Düsen hatten einen Durchmesser von 2 beziehungsweise 3 mm.

Der Wassergehalt der Extrudate lag bei kleiner 3 Gew.-%..

Die Bestimmung des amorphen Zustands erfolgte mittels Polarisationsmikroskopie.

Allgemeines zur Herstellvorschrift Polymere E-G

Die Polymerisation erfolgte in einem 2 I -Reaktor mit Ankerrührer. Die erhaltenen Polymerlösungen wurden durch Gefriertrocknung in Pulver überführt.
VE-Wasser: vollentsalztes Wasser

### Herstellung von Polymer E

Copolymer aus 90 Gew.-% Acrylsäure und 10 gew.-% 2-Sulfoethylmethacrylat

Monomermenge: 240.0g Ansatzgröße: 802.8g

### Vorlage Gesamt: 320.0g

| | |
|---|---|
| 320.0g | VE-Wasser |

### Zulauf 1 Gesamt: 276.0g

| | |
|---|---|
| 36.0g | VE-Wasser |
| 24.0g | 2-Sulfoethylmethacrylat |
| 216.0g | Acrylsäure |

### Zulauf 2 Gesamt: 124. 4g

| | |
|---|---|
| 122.0g | VE-Wasser |
| 2.4g | Natriumhypophosphit monohydrat |

### Zulauf 3 Gesamt: 82.4g

| | |
|---|---|
| 2.4g | Natriumperoxodisulfat |
| 80.0g | VE-Wasser |

Die Vorlage wurde bei 120 Upm unter Stickstoffspülung auf 90°C Innentemperatur aufgeheizt.

Wenn 90°C Innentemperatur erreicht waren, wurden die Zuläufe 1 und 2 in 4 Stunden und Zulauf 3 in 4 Stunden 15 Minuten zudosiert. Nach Ende von Zulauf 3 liess man den Ansatz noch 1 Stunde bei 90°C nachpolymerisieren. Anschliessend wurde der Ansatz auf Raumtemperatur abgekühlt. Man erhielt eine leicht viskose, klare, gelbe Lösung mit einem

Feststoffgehalt von 29,9 Gew.% und einem K-Wert 46.

### Herstellung von Polymer F

Copolymer aus 90 Gew.% Acrylsäure und 10 Gew.% Itaconsäure

Monomermenge: 560.0g Ansatzgröße: 1651.2g

Zuordnung Menge Einsatzstoff

Vorlage Gesamt: 682.4g

| | |
|---|---|
| 56.0g | Itaconsäure |
| 0.6g | Eisen-II-sulfat heptahydrat 1.0%ig in Wasser |
| 611.8g | VE-Wasser |
| 14.0g | Von Zulauf 2 |

### Zulauf 1 Gesamt: 702.8g

| | |
|---|---|
| 504.0g | Acrylsäure |
| 196.0g | VE-Wasser |
| 2.8g | Natriumhypophosphit monohydrat |

### Zulauf 2 Gesamt: 280.0g

| | |
|---|---|
| 56.0g | Wasserstoffperoxid 30.0%ig |
| 224.0g | VE-Wasser |

Die Vorlage wurde bei 120UPM unter Stickstoffbegasung auf 95°C Innentemperatur aufgeheizt. Wenn 95°C Innentemperatur erreicht waren, wurde Zulauf 1 in 5 Stunden und Zulauf 3 in 6 Stunden zudosiert. Nach Ende von Zulauf 2 polymerisiert der Ansatz noch 2h bei 95°C nach. Anschliessend wurde der Ansatz auf Raumtemperatur abgekühlt. Man erhielt eine klare, farblose, leicht viskose Lösung mit einem Feststoffgehalt von 35,0 Gew.-% und einem K-Wert 55.

### Herstellung von Polymer G

Copolymer aus 80 Gew.% Acrylsäure, 10 Gew. % 2-Acrylamido-2- methylpropansulfonsäure und 10 Gew.% Acrylamid

Monomermenge: 240.0g Ansatzgröße: 800.4g

| | |
|---|---|
| Menge | Einsatzstoff |
| Vorlage Gesamt: | 296.0g |
| 296.0g | VE-Wasser |

### Zulauf 1 Gesamt: 240.0g

| | |
|---|---|
| 192.0g | Acrylsäure |
| 24.0g | 2-Acrylamido-2- methylpropansulfonsäure |
| 24.0g | VE-Wasser |

### Zulauf 2 Gesamt: 182.0g

| | |
|---|---|
| 158.0g | VE-Wasser |
| 24.0g | Acrylamid |

### Zulauf 3 Gesamt: 82.4g

| | |
|---|---|
| 2.4g | Natriumperoxodisulfat |
| 80.0g | VE-Wasser |

Die Vorlage wurde bei 120 Upm unter Stickstoffbegasung auf 90°C Innentemperatur aufgeheizt. Wenn 90°C Innentemperatur erreicht waren, wurden die Zuläufe 1 und 2 in 4 Stunden und Zulauf 3 in 4 Stunden 15Minuten zudosiert. Nach Ende von Zulauf 3 liess man den Ansatz noch 1 h bei 90°C nach polymerisieren. Anschliessend wurde der Ansatz auf Raumtemperatur abgekühlt. Man erhielt eine klare, viskose Lösung mit einem Feststoffgehalt von 30,8 Gew.-% und einem K-Wert 74 .

### Herstellung von Polymer H

Copolymer aus 89,2 Gew.% Acrylsäure und 10,8 Gew.% 2-Acrylamido-2- methylpropansulfonsäure

Monomermenge: 242.1g Ansatzgröße: 802.5g

| | |
|---|---|
| Menge | Einsatzstoff |
| Vorlage Gesamt: | 320.0 g |
| 320.0g | VE-Wasser |

### Zulauf 1 Gesamt: 400.1 g

| | |
|---|---|
| 158.0g | VE-Wasser |
| 26.1g | 2-Acrylamido-2- methylpropansulfonsäure |
| 216.0g | Acrylsäure |

### Zulauf 2 Gesamt: 82.4 g

| | |
|---|---|
| 2.4g | Natriumperoxodisulfat |
| 80.0g | VE-Wasser |

Die Vorlage wurde bei 120 Upm gerührt und unter Stickstoffspülung auf 90°C Innentemperatur aufgeheizt. Wenn 90°C Innentemperatur erreicht waren, wurde Zulauf 1 in 4 Stunden und Zulauf 2 in 4 Stunden 15 Minuten zudosiert. Nach Ende von Zulauf 2 liess man den Ansatz noch 1 Stunde bei 90°C nach polymerisieren. Anschliessend wurde der Ansatz auf Raumtemperatur abgekühlt. Man erhielt eine klare, viskose, farblose Lösung mit einem Feststoffgehalt von 32,2 Gew.-% und einem K-Wert 82.

### Herstellung von polymeren Salzen

### Beispiel 1

### Löslichkeiten von Loperamid in wässrigen Polymerlösungen

1,0 g Loperamid (freie Base) wurde zu 100,0 ml wässriger Polymerlösung gegeben und 72h gerührt. Dabei bildete sich das polymeren Salz aus bis zu seiner Sättigungslöslichkeit in Wasser. Die nicht gelöste Menge an Loperamid wurde abfiltriert. Im Filtrat wurde der Gehalt an Loperamid per HPLC bestimmt

Zum Vergleich wurde die Sättigungslöslichkeit von Loperamid in entsalztem Wasser bestimmt sowie in Salzsäure. Bei letzterem wird das entsprechende Loperamid-Hydrochlorid gebildet.

| Polymerkonzentration in Wasser [Gew.-%] | Polymer | Konzentration gelöstes Loperamid [mg/ml] | pH-Wert der filtrierten Lösung |
|---|---|---|---|
| 0 | Wasser | < 0,0170 | |
| 10 | A | 6,2 | 2,5 |
| 20 | A | 7,6 | |
| 10 | B | 3,9 | 2,0 |
| 20 | D | 5,9 | 1,9 |
| Salzsäure 10% | - | <0,0149 | |

Die Löslichkeit von Loperamid in 20%iger Lösung von Polymer A ist gegenüber Wasser mindestens um den Faktor 447 und gegenüber 10 gew.-%iger wässriger Salzsäurelösung um den Faktor 510 verbessert.

### Beispiel 2

### Löslichkeiten von Haloperidol in wässrigen Polymerlösungen

1,0 g Haloperidol (freie Base) wurde zu 100,0 ml wässriger Polymerlösung gegeben und 72h gerührt. Dabei bildete sich das polymeren Salz aus bis zu seiner Sättigungslöslichkeit in Wasser. Die nicht gelöste Menge an Haloperidol wurde abfiltriert. Im Filtrat wurde der Gehalt an Haloperidol per HPLC bestimmt

Zum Vergleich wurde die Sättigungslöslichkeit von Haloperidol in entsalztem Wasser bestimmt sowie in Salzsäure. Bei letzterem wird das entsprechende Haloperidol - Hydrochlorid gebildet.

Zum Nachweis, dass die Löslichkeitsverbesserung tatsächlich auf einer Salzbildung und nicht auf einer solubilisierenden Wirkung beruht, wurde eine 20%ige Lösung von Polymer A mit NaOH auf pH 10 gestellt und damit die Sättigungslöslichkeit bestimmt.

| Polymerkonzentration in Wasser [Gew.-%] | Polymer | Konzentration gelöstes Haloperidol [mg/ml] | pH-Wert der filtrierten Lösung |
|---|---|---|---|
| 0 | Wasser | < 0,0178 | 7,5 |
| 20 | A | 10,3 | 3,0 |
| 20 | C | 7,7 | 3,3 |
| 10 | D | 5,2 | 3,2 |
| 20 | D | 15,1 | 3,0 |
| 20 | Lösung plus NaOH bis pH-Wert 10 | <0,074 | 10,1 |
| Salzsäure 10% | - | <0,0160 | |

Die Löslichkeit von Haloperidol in 20%iger Polymer A-Lösung ist gegenüber Wasser mindestens um den Faktor 579 und gegenüber 10gew.-%iger wässriger Salzsäurelösung um den Faktor 644 verbessert.

### Beispiel 3

### Löslichkeiten von Cinnarizin in wässrigen Polymerlösungen

1,0 g Cinnarizin (freie Base) wurde zu 100,0 ml wässriger Polymerlösung gegeben und 72h gerührt. Dabei bildete sich das polymeren Salz aus bis zu seiner Sättigungslöslichkeit in Wasser. Die nicht gelöste Menge an Cinnarizin wurde abfiltriert. Im Filtrat wurde der Gehalt an Cinnarizin per HPLC bestimmt

Zum Vergleich wurde die Sättigungslöslichkeit von Cinnarizin in entsalztem Wasser bestimmt sowie in Salzsäure. Bei letzterem wird das entsprechende Cinnarizin - Hydrochlorid gebildet.

| Polymerkonzentration in Wasser [Gew.-%] | Polymer | Konzentration gelöstes Cinnarizin [mg/ml] |
|---|---|---|
| 0 | Wasser | 0,01 |
| 10 | A | 2,8 |
| 20 | A | 9,6 |
| 10 | C | 4,0 |
| 20 | C | 10,1 |
| 10 | D | 4,1 |
| 20 | D | 10,5 |
| Salzsäure 10% | - | <0,79 |

Die Löslichkeit von Cinnarizin in 20%iger Polymer A-Lösung ist gegenüber Wasser mindestens um den Faktor 480 und gegenüber 10gew.-%iger wässriger Salzsäurelösung um den Faktor 13 verbessert.

### Beispiel 4

### Herstellung eines Salzes aus Loperamid und Polymer A durch Sprühtrocknung

36g Loperamid wurden in 1464g einer 20%igen wässrigen Polymer A- Lösung unter Rühren 2h dispergiert. Hierbei ging ein Teil des Wirkstoffes bereits in Lösung. Diese Dispersion wurde kurz vor der Sprühtrocknung kurzfristig auf 90°C erhitzt, wobei sich das Loperamid vollständig löste. Direkt anschließend erfolgte die Sprühtrocknung bei folgenden Bedingungen: Eingangsgastemperatur: 200°C
Ausgangsgastemperatur: 103°C
Sprührate: 9,5g/min
Es wurde ein trockenes Pulver erhalten.

Produkteigenschaften:
Restfeuchte: 2,1 % (g/g)
Löslichkeit des polymeren Loperamidsalzes in
Wasser gemessen als gelöstes Loperamid: 17,0 mg/ml

### Beispiel 5

### Herstellung eines Salzes aus Loperamid und Polymer B durch Sprühtrocknung

36g Loperamid wurden in 1464g einer 20%igen wässrigen Polymer B- Lösung unter Rühren 2h dispergiert. Hierbei ging ein Teil des Wirkstoffes bereits in Lösung. Diese Dispersion wurde kurz vor der Sprühtrocknung kurzfristig auf 90°C erhitzt, wobei sich das Loperamid vollständig löste. Direkt anschließend erfolgte die Sprühtrocknung bei folgenden Bedingungen: Eingangsgastemperatur: 168°C
Ausgangsgastemperatur: 77°C
Sprührate: 9,5g/min
Es wurde ein trockenes Pulver erhalten.

Produkteigenschaften:
Restfeuchte: 6,1 % (g/g)
Löslichkeit des polymeren Loperamidsalzes in
Wasser gemessen als gelöstes Loperamid: 13,3 mg/ml

### Beispiel 6

### Herstellung eines Salzes aus Haloperidol und Polymer A durch Sprühtrocknung

54g Haloperidol wurden in 1576g einer 20%igen wässrigen Polymer A- Lösung unter Rühren 2h dispergiert. Hierbei ging ein Teil des Wirkstoffes bereits in Lösung. Diese Dispersion wurde kurz vor der Sprühtrocknung kurzfristig auf 90°C erhitzt, wobei sich das Haloperidol vollständig löste. Direkt anschließend erfolgte die Sprühtrocknung bei folgenden Bedingungen: Eingangsgastemperatur: 193°C
Ausgangsgastemperatur: 85°C
Sprührate: 12,5g/min
Es wurde ein trockenes Pulver erhalten.

Produkteigenschaften:
Restfeuchte: 2,0% (g/g)
Löslichkeit des polymeren Haloperidolsalzes in
Wasser gemessen als gelöstes Haloperidol: 34,1 mg/ml

### Beispiel 7

### Herstellung eines Salzes aus Haloperidol und Polymer B-Lösung durch Sprühtrocknung

58g Haloperidol wurden in 1692g einer 20%igen wässrigen Polymer B- Lösung unter Rühren 2h dispergiert. Hierbei ging ein Teil des Wirkstoffes bereits in Lösung. Diese Dispersion wurde kurz vor der Sprühtrocknung kurzfristig auf 90°C erhitzt, wobei sich das Haloperidol vollständig löste. Direkt anschließend erfolgte die Sprühtrocknung bei folgenden Bedingungen: Eingangsgastemperatur: 173°C
Ausgangsgastemperatur: 91 °C
Sprührate: 8,7g/min
Es wurde ein trockenes Pulver erhalten.

Produkteigenschaften:
Restfeuchte: 5,7% (g/g)
Löslichkeit des polymeren Haloperidolsalzes in
Wasser gemessen als gelöstes Haloperidol: 17,3 mg/ml

### Beispiel 8

### Herstellung eines Salzes aus Cinnarizin und Polymer A durch Sprühtrocknung

24,5g Cinnarizin wurden in 1725,5g einer 20%igen wässrigen Polymer A- Lösung unter Rühren 2h dispergiert. Hierbei ging ein Teil des Wirkstoffes bereits in Lösung. Diese Dispersion wurde kurz vor der Sprühtrocknung kurzfristig auf 90°C erhitzt, wobei sich das Cinnarizin vollständig löste. Direkt anschließend erfolgte die Sprühtrocknung bei folgenden Bedingungen: Eingangsgastemperatur: 177°C
Ausgangsgastemperatur: 95°C
Sprührate: 17,6g/min
Es wurde ein trockenes Pulver erhalten.

Produkteigenschaften:
Restfeuchte: 2,1% (g/g)
Löslichkeit des polymeren Cinnarizinsalzes in
Wasser gemessen als gelöstes Cinnarizin: 7,9 mg/ml

### Beispiel 9

### Herstellung eines Salzes aus Loperamid und Polymer A durch Lösen in einem organischen Lösungsmittel und anschließendes Abdampfen

5g Loperamid wurden in 150g einer 30%igen Lösung von Polymer A in Ethanol unter Rühren 2h gelöst. Diese Lösung wurde am Rotationsverdampfer bis zur Trockene eingedampft. Der erhaltene Feststoff wurde anschließend zu einem Pulver gemahlen.

Produkteigenschaften:
Restfeuchte: 1,4 % (g/g)
Löslichkeit des polymeren Loperamidsalzes in Wasser gemessen als gelöstes Loperamid: 15,9 mg/ml

### Beispiel 10

### Herstellung eines Salzes aus Haloperidol und Polymer A durch Lösen in einem organischen Lösungsmittel und anschließendes Abdampfen

10g Haloperidol wurden in 150g einer 30%igen Lösung von Polymer A in Ethanol unter Rühren 2h gelöst. Diese Lösung wurde am Rotationsverdampfer bei 80°C bis zur Trockene eingedampft. Der erhaltene Feststoff wurde anschließend zu einem Pulver gemahlen.

Produkteigenschaften:
Restfeuchte: 1,6% (g/g)
Löslichkeit des polymeren Haloperidolsalzes in
Wasser gemessen als gelöstes Haloperidol: 25,4 mg/ml

### Beispiel 11

### Herstellung eines Salzes aus Loperamid und Polymer A durch Extrusion

500g Loperamid und 4500g Polymer A wurden zunächst in einem Mischer homogen vermischt und anschließend in einem Zweischneckenextruder bei 130°C extrudiert. Die Verweilzeit betrug 3,5 min. Der erhaltenen Stränge von 2mm Dicke wurden anschlie-βend zu einem Pulver gemahlen.

Produkteigenschaften:
Restfeuchte: 1,4% (g/g)
Löslichkeit des polymeren Loperamidsalzes in
Wasser gemessen als gelöstes Loperamid: 16,2 mg/ml

### Beispiel 12

### Herstellung eines Salzes aus Cinnarizin und Polymer A durch Extrusion

700g Cinnarizin und 4500g Polymer A wurden zunächst in einem Mischer homogen vermischt und anschließend in einem Zweischneckenextruder bei 135°C extrudiert. Die Verweilzeit betrug 3,5 min. Die erhaltenen Stränge von 2mm Dicke wurden anschlie-βend zu einem Pulver gemahlen.

Produkteigenschaften:
Restfeuchte: 1,6% (g/g)
Löslichkeit des polymeren Cinnarizinsalzes in
Wasser gemessen als gelöstes Cinnarizin: 7,2 mg/ml

### Bespiel 13

### Herstellung eines Salzes aus Famotidin und Polymer G durch Extrusion

500g Famotidin, 3500g Polymer 3 und 800g Poloxamer 188 wurden zunächst in einem Mischer homogen vermischt und anschließend in einem Zweischneckenextruder bei 140°C extrudiert. Die Verweilzeit betrug 4min. Die erhaltenen 3mm dicken Stränge wurden anschließend zu einem Pulver gemahlen.

Produkteigenschaften:
Restfeuchte: 0,5% (g/g)
Das gemahlene Pulver war amorph. Löslichkeit des polymeren Famotidinsalzes in Wasser gemessen als gelöstes Famotidin: 45,3mg/ml

### Beispiel 14

### Herstellung eines Salzes aus Famotidin und Polymer Fdurch Extrusion

500g Famotidin und 4500 g Polymer F wurden zunächst in einem Mischer homogen vermischt und anschließend in einem Zweischneckenextruder bei 130°C extrudiert. Über eine zweite Dosierstelle wurden 750g Wasser zugeführt. Die Verweilzeit betrug 2 min. Die erhaltenen Stränge von 3 mm Dicke wurden granuliert, getrocknet und anschließend zu einem Pulver gemahlen.

Produkteigenschaften:
Restfeuchte: 2,5% (g/g)
Das gemahlene Pulver war amorph. Löslichkeit des polymeren Famotidinsalzes in Wasser gemessen als gelöstes Famotidin: 22,5mg/ml

### Beispiel 15

### Herstellung eines Salzes aus Cinnarizin und Polymer H durch Extrusion

700g Cinnarizin, 3000g Polymer H und 1000g PEG 1500 wurden zunächst in einem Mischer homogen vermischt und anschließend in einem Zweischneckenextruder bei 140°C extrudiert. Die Verweilzeit betrug 2min. Die erhaltenen 2mm dicken Stränge wurden anschließend zu einem Pulver gemahlen.

Produkteigenschaften:
Restfeuchte: 0,5% (g/g)
Das gemahlene Pulver war amorph. Löslichkeit des polymeren Cinnarizinsalzes in Wasser gemessen als gelöstes Cinnarizin: 9,3mg/ml

### Beispiel 16

### Herstellung eines Salzes aus Cinnarizin und Polymer E durch Extrusion

600g Cinnarizin und 4500g Polymer E wurden mit zwei Pulver-dosierungen simultan im Verhältnis 1:10 in den Zweischneckenextruder hineindosiert. Über eine weitere Dosierstelle wurden 650g Ethanol zugefahren. Die Extrusion erfolgte bei 125°C, wobei die Verweilzeit 2min betrug. Die erhaltenen 2mm dicken Stränge wurden anschließend zu einem Pulver gemahlen.

Produkteigenschaften:
Restfeuchte: 1,7% (g/g)
Das gemahlene Pulver war amorph. Löslichkeit des polymeren Cinnarizinsalzes in Wasser gemessen als gelöstes Cinnarizin: 5,7mg/ml

### Beispiel 17

### Herstellung eines Salzes aus Cinnarizin und Polymer Edurch Extrusion

600 g Cinnarizin und 4000 g Polymer E wurden zunächst in einem Mischer homogen vermischt und anschließend in den Zweischneckenextruder hineindosiert. Über eine weitere Dosierstelle wurden 800 g Propylenglykol zugefahren. Die Extrusion erfolgte bei 115°C, wobei die Verweilzeit 2 min betrug. Die erhaltenen 2mm dicken Stränge wurden anschließend zu einem Pulver gemahlen.

Produkteigenschaften:
Restfeuchte: 1,4% (g/g)
Das gemahlene Pulver war amorph. Löslichkeit des polymeren Cinnarizinsalzes in Wasser gemessen als gelöstes Cinnarizin: 8,0mg/ml

### Beispiel 18

### Herstellung von Tabletten mit einem polymeren Salz aus Loperamid und Polymer A

17g polymeres Loperamidsalz aus Beispiel 11 wurden mit 150g mikrokristalliner Cellulose, 118 g Dicalciumphosphat, 12g Crospovidon und 3g Magnesiumstearat gemischt und auf einer Excentertablettenpresse zu Tabletten mit folgenden Eigenschaften verpresst.
Durchmesser: 10mm
Gewicht: 300mg
Bruchfestigkeit: 60N
Zerfall: 33s
Wirkstofffreisetzung in Wasser: 99% nach 15 min

### Beispiel 19

### Herstellung von Tabletten mit einem polymeren Salz aus Haloperidol und Polymer A

65g polymeres Haloperidolsalz aus Beispiel 4 wurden mit 110g mikrokristalliner Cellulose, 110 g Dicalciumphosphat, 12g Crospovidon und 3g Magnesiumstearat gemischt und auf einer Rundläufertablettenpresse zu Tabletten mit folgenden Eigenschaften verpresst.
Durchmesser: 10mm
Gewicht: 300mg
Bruchfestigkeit: 80N
Zerfall: 68s
Wirkstofffreisetzung in Wasser: 99% nach 15 min

## Patentansprüche

1. Polymere, wasserlösliche Salze von in Wasser schwerlöslichen Arzneistoffen, bestehend wasserlöslichen Polymer mit Anioncharakter, wobei das Polymer mit Anioncharakter einen Anteil an carboxylgruppen tragenden Monomeren von mindestens 40 Gew.-% und in 1 gew.-%iger wässriger Lösung einen K-Wert nach Fikentscher von kleiner 90 aufweist und die Wasserlöslichkeit im pH-Bereich von 3 bis 11 größer 10 % (m/m) beträgt, und einem schwerlöslichen Arzneistoff mit Kationcharakter, wobei der Arzneistoff in nicht geladener Form oder als Hydrochlorid eine Löslichkeit von kleiner 0.1 % (m/m) in Wasser, künstlichem Darmsaft oder Magensaft aufweiset.

2. Polymeres Salz nach Anspruch 1, wobei das Polymer mit Anioncharakter nicht gelbildend ist.

3. Polymeres Salz nach einem der Ansprüche 1oder 2, wobei der Arzneistoff mindestens eine und höchstens zwei zur Salzbildung befähigten Gruppen aufweist.

4. Polymeres Salz nach einem der Ansprüche 1 bis 3, wobei das polymere Arzneistoffsalz eine höhere Wasserlöslichkeit als der Arzneistoff und das entsprechende Hydrochlorid des Arzneistoffes aufweist.

5. Polymeres Salz nach einem der Ansprüche 1 bis 4, wobei das Polymer mit Anioncharakter in 1 gew.-%iger wässriger Lösung einen K-Wert nach Fikentscher von kleiner 50 aufweist.

6. Polymeres Salz nach einem der Ansprüche 1 bis 5, wobei das Polymer mit Anioncharakter neben Carboxylgruppen Phosphonsäure- oder Sulfonsäuregruppen oder eine Kombination von diesen enthalten kann.

7. Polymeres Salz nach einem der Ansprüche 1 bis 6, wobei das Polymer mit Anioncharakter als carboxylgruppentragende Monomere Acrylsäure, Methacrylsäure oder Maleinsäure oder Mischungen davon enthält.

8. Polymeres Salz nach einem der Ansprüche 1 bis7, enthaltend als carboxylgruppentragenden Monomere eine Kombination von Itaconsäure mit Acrylsäure, Methacrylsäure oder Maleinsäure.

9. Polymeres Salz nach einem der Ansprüche 1 bis8, enthaltend 1 bis 20 Gew.-% an Sulfonsäuregruppen tragenden Monomeren.

10. Polymeres Salz nach einem der Ansprüche 1 bis9, enthaltend 1 bis 20 Gew.-% an neutralen Comonomeren.

11. Feste Darreichungsform enthaltend polymere Salze nach einem der Ansprüche 1 bis10.

12. Feste Darreichungsform nach Anspruch11, hergestellt durch Verpressung.

13. Verfahren zur Herstellung polymerer, wasserlöslicher Salze von Arzneistoffen gemäß einem der Ansprüche 1 bis 10, **dadurch gekennzeichnet, dass** ein wasserlösliches Polymer mit Anionencharakter, wobei das Polymer mit Anioncharakter einen Anteil an carboxylgruppen tragenden Monomeren von mindestens 40 Gew.-% und in 1 gew.-%iger wässriger Lösung einen K-Wert nach Fikentscher von kleiner 90 aufweist und die Wasserlöslichkeit im pH-Bereich von 3 bis 11 größer 10 % (m/m) beträgt, und ein schwerlöslicher Arzneistoff mit Kationcharakter, wobei der Arzneistoff in nicht geladener Form oder als Hydrochlorid eine Löslichkeit von kleiner 0.1 % (m/m) in Wasser, künstlichem Darmsaft oder Magensaft aufweist, in einem Lösungsmittel aufgelöst werden und das polymere Arzneistoffsalz aus der Lösung isoliert wird.

14. Verfahren nach Anspruch13, **dadurch gekennzeichnet, dass** das polymere Arzneistoffsalz durch Trocknung der Lösung isoliert wird.

15. Verfahren nach Anspruch13, **dadurch gekennzeichnet, dass** das polymere Arzneistoffsalz aus der Lösung durch Präzipitation infolge einer Abkühlung oder einer Zugabe eines Nichtlösungsmittels gewonnen wird.

16. Verfahren zur Herstellung polymerer, wasserlöslicher Salze von Arzneistoffen gemäß einem der Ansprüche 1 bis 10, **dadurch gekennzeichnet, dass** die Herstellung durch gemeinsame Verarbeitung der Polymere mit Anioncharakter und Wirkstoff in einem Extruder erfolgt.

## Claims

1. A polymeric water-soluble salt of medicaments that are sparingly soluble in water, consisting of a water-soluble polymer with anion character, where the polymer with anion character has a fraction of carboxyl-group-carrying monomers of at least 40% by weight in 1% strength by weight aqueous solution has a K value in accordance with Fikentscher of less than 90 and the solubility in water in the pH range from 3 to 11 is greater than 10% (m/m), and a sparingly soluble medicament with cation character, where the medicament, in uncharged form or as hydrochloride, has a solubility of less than 0.1% (m/m) in water, artificial intestinal juice or gastric juice.

2. The polymeric salt according to claim 1, where the polymer with anion character is not gel-forming.

3. The polymeric salt according to either of claims 1 and 2, where the medicament has at least one and at most two groups capable of salt formation.

4. The polymeric salt according to any one of claims 1 to 3, where the polymeric medicament salt has a higher solubility in water than the medicament and the corresponding hydrochloride of the medicament.

5. The polymeric salt according to any one of claims 1 to 4, where the polymer with anion character in 1% strength by weight aqueous solution has a K value in accordance with Fikentscher of less than 50.

6. The polymeric salt according to any one of claims 1 to 5, where the polymer with anion character can comprise, besides carboxyl groups, phosphoric acid or sulfonic acid groups or a combination of these.

7. The polymeric salt according to any one of claims 1 to 6, where the polymer with anion character comprises, as carboxyl-group-carrying monomers, acrylic acid, methacrylic acid or maleic acid or mixtures thereof.

8. The polymeric salt according to any one of claims 1 to 7, comprising, as carboxyl-group-carrying monomers, a combination of itaconic acid with acrylic acid, methacrylic acid or maleic acid.

9. The polymeric salt according to any one of claims 1 to 8, comprising 1 to 20% by weight of monomers carrying sulfonic acid groups.

10. The polymeric salt according to any one of claims 1 to 9, comprising 1 to 20% by weight of neutral comonomers.

11. A solid administration form comprising polymeric salts according to any one of claims 1 to 10.

12. The solid administration form according to claim 11, produced by compression.

13. A method for producing polymeric, water-soluble salts of medicaments according to any one of claims 1 to 10, wherein a water-soluble polymer with anion character, where the polymer with anion character has a fraction of carboxyl-group-carrying monomers of at least 40% by weight and in 1% strength by weight aqueous solution has a K value in accordance with Fikentscher of less than 90 and the solubility in water in the pH range from 3 to 11 is greater than 10% (m/m), and a sparingly soluble medicament with cation character, where the medicament, in uncharged form or as hydrochloride, has a solubility of less than 0.1% (m/m) in water, artificial intestinal juice or gastric juice, are dissolved in a solvent and the polymeric medicament salt is isolated from the solution.

14. The method according to claim 13, wherein the polymeric medicament salt is isolated by drying the solution.

15. The method according to claim 13, wherein the polymeric medicament salt is obtained from the solution by precipitation as a result of cooling or an addition of a nonsolvent.

16. A method for producing polymeric, water-soluble salts of medicaments according to any one of claims 1 to 10, wherein the preparation takes place by combined processing of the polymers with anion character and active ingredient in an extruder.

## Revendications

1. Sels polymères, solubles dans l'eau de médicaments peu solubles dans l'eau, constitués d'un polymère soluble dans l'eau doté d'un caractère anionique, le polymère doté d'un caractère anionique présentant une proportion de monomères portant des groupes carboxyle d'au moins 40% en poids et une valeur K selon Fikentscher dans une solution aqueuse à 1% en poids inférieure à 90 et la solubilité dans l'eau dans la plage de pH de 3 à 11 étant supérieure à 10% (m/m), et d'un médicament peu soluble doté d'un caractère cationique, le médicament sous forme non chargée ou sous forme de chlorhydrate présentant une solubilité inférieure à 0,1% (m/m) dans l'eau, le suc intestinal ou gastrique synthétique.

2. Sel polymère selon la revendication 1, le polymère doté d'un caractère anionique n'étant pas gélifiant.

3. Sel polymère selon l'une quelconque des revendications 1 ou 2, le médicament présentant au moins un et au plus deux groupes aptes à une formation de sel.

4. Sel polymère selon l'une quelconque des revendications 1 à 3, le sel médicamenteux polymère présentant une solubilité dans l'eau supérieure à celle du médicament et du chlorhydrate correspondant du médicament.

5. Sel polymère selon l'une quelconque des revendications 1 à 4, le polymère doté d'un caractère anionique présentant une valeur K selon Fikentscher dans une solution aqueuse à 1% en poids inférieure à 50.

6. Sel polymère selon l'une quelconque des revendications 1 à 5, le polymère doté d'un caractère anionique pouvant contenir, en plus des groupes carboxyle, des groupes acide phosphorique ou des groupes acide sulfonique ou une combinaison de ceux-ci.

7. Sel polymère selon l'une quelconque des revendications 1 à 6, le polymère doté d'un caractère anionique contenant comme monomères portant des groupes carboxyle l'acide acrylique, l'acide méthacrylique ou l'acide maléique ou des mélanges de ceux-ci.

8. Sel polymère selon l'une quelconque des revendications 1 à 7, contenant comme monomères portant des groupes carboxyle une combinaison d'acide itaconique avec de l'acide acrylique, de l'acide méthacrylique ou de l'acide maléique.

9. Sel polymère selon l'une quelconque des revendications 1 à 8, contenant 1 à 20% en poids de monomères portant des groupes acide sulfonique.

10. Sel polymère selon l'une quelconque des revendications 1 à 9, contenant 1 à 20% en poids de comonomères neutres.

11. Forme d'administration solide contenant des sels polymères selon l'une quelconque des revendications 1 à 10.

12. Forme d'administration selon la revendication 11, préparée par pressage.

13. Procédé pour la préparation de sels polymères, solubles dans l'eau, de médicaments selon l'une quelconque des revendications 1 à 10, **caractérisé en ce qu'**on dissout, dans un solvant, un polymère soluble dans l'eau doté d'un caractère anionique, le polymère doté d'un caractère anionique présentant une proportion de monomères portant des groupes carboxyle d'au moins 40% en poids et une valeur K selon Fikentscher dans une solution aqueuse à 1% en poids inférieure à 90 et la solubilité dans l'eau dans la plage de pH de 3 à 11 étant supérieure à 10% (m/m), et un médicament peu soluble doté d'un caractère cationique, le médicament sous forme non chargée ou sous forme de chlorhydrate présentant une solubilité inférieure à 0,1% (m/m) dans l'eau, le suc intestinal ou gastrique synthétique et le sel médicamenteux polymère est isolé de la solution.

14. Procédé selon la revendication 13, **caractérisé en ce que** le sel médicamenteux polymère est isolé par séchage de la solution.

15. Procédé selon la revendication 13, **caractérisé en ce que** le sel médicamenteux polymère est obtenu de la solution par précipitation suite à un refroidissement ou une addition d'un non-solvant.

16. Procédé pour la préparation de sels polymères, solubles dans l'eau, de médicaments selon l'une quelconque des revendications 1 à 10, **caractérisé en ce que** la préparation est réalisée par une transformation commune du polymère doté d'un caractère anionique et de la substance active dans une extrudeuse.
